Europäisches Patentamt

European Patent Office

Office européen des brevets

(11) Publication number: 0 355 009 A1

## (12) EUROPEAN PATENT APPLICATION

(21) Application number: 89114623.5

(22) Date of filing: 08.08.89

(51) Int. Cl.4: A61K 31/635 , A61K 47/00 , A61K 9/127

(30) Priority: 18.08.88 DE 3828044

(43) Date of publication of application:
21.02.90 Bulletin 90/08

(84) Designated Contracting States:
AT BE CH DE ES FR GB GR IT LI LU NL SE

(71) Applicant: Minninger, Konrad
Hattinger Strasse 45
D-5830 Schwelm(DE)

(72) Inventor: Minninger, Konrad
Hattinger Strasse 45
D-5830 Schwelm(DE)
Inventor: Tang, David, Dr.
Hauptstrasse 304
D-4690 Herne 2(DE)
Inventor: Oberhagemann, Rainer
Hauptstrasse 304
D-4690 Herne 2(DE)

(74) Representative: Redies, Bernd, Dr. rer. nat. et al
COHAUSZ & FLORACK Patentanwaltsbüro
Schumannstrasse 97 Postfach 14 01 20
D-4000 Düsseldorf 1(DE)

(54) Silver sulfadiazine containing pharmaceutical product for the local external therapy.

(57) The present invention is related to a silver sulfadiazine containing pharmaceutical product for the local, external therapy of herpes labialis, herpes genitalis, herpes corporis, herpes zoster, chicken-poxs and eczema herpaticatum in humans. The silver sulfadiazine containing pharmaceutical product contains silver sulfadiazine in combination with one or several non-ionic surfactants in a readily spreadable base material compatible to the human skin, in particular in an aqueous gel. The pharmaceutical product of the invention consists in particular of 0.05 to 10 % by weight of silver sulfadiazine, 0.3 to 23 % by weight of the non-ionic surfactant or a mixture of several such products and 99.65 to 67.00 % by weight of the readily spreadable base compatible to human skin. Thus, the total amount of the above components is 100 % by weight and represents the pharmaceutical preparation according to the present invention.

# Silver sulfadiazine containing pharmaceutical product for the local external therapy

The present invention refers to a silver sulfadiazine containing pharmaceutical product for the local external therapy of herpes labialis, herpes genitalis, herpes corporis, herpes zoster, chicken-poxs and eczema herpaticatum in humans.

It is known for instance from R.. Zimmermann and W. Kräuter, "Krankenhauspharmazie", Vol. 7, No. 11 (1986) that the silver salt of sulfadiazine (i.e. silver sulfadiazine) is a non-toxic antiseptic active on the surface of the human skin. This compound in combination with cerium-(III)-nitrate in a pastelike prepartion is externally used for the preliminary treatment of wound infections after burns. It has been further proposed to use silver sulfadiazine in a soft creme miscible with water for the treatment of herpes zoster (see L.F. Montes, G. Muchinik, Ch. L. Fox, "Response of Varicella zoster Virus and Herpes Zoster to Silver Sulfadiazine"; Cutis; Vol. 38 (1986) pgs. 363 to 365). Silver sulfadiazine is a compound slowly dissociating wherein the $Ag^+$-cation is the active principle. This compound produces an inactivating activity to the cell membranes, it interferes with the synthesis of DNA (i.e. desoxyribonucleic acid) (suppression of virus maturing) and prevents the virus dissemination into the central nervous system.

There are known several other antivirally active compounds which also have been used against herpes viruses. They partly interfere with the synthesis of DNA in the same way as silver sulfadiazine or they are active against herpes viruses in other ways. Some attack the virus cell, some attack their propagation by other mechanisms. Zinc sulphate ($ZnSO_4$) is used in microcutaneous infections caused by herpes simplex viruses (HSV) and is characterized by the absence of any side reactions, as has been determined up to now (see R. Braun, Apotheker Journal 7/88, pgs. 22 to 28). Various surfactants also have shown a high inactivating activity against HSV. For instance, cationic detergents based upon quaternary ammonium compounds are known as highly active inactivators of HSV (see f.i. M. Klein and A. De Forest, Soap Chem. Spec. 39 (1963), pgs. 70 to 72). Non-ionic surfactants too show differing effectivity in inactivation of HSV, in particular non-ionic surfactants having only ether groupments between the hydrocarbon radicals of the molecule. Some of these latter products are in trade in various intravaginally used anticonceptiva.

Some known antivirally active pharmaceutical preparations used already over prolonged periods of time show signs of resistency.

Thus, it is an object of the present invention to produce a pharmaceutical product for the treatment of the above herpes deseases which is securely active against herpes viruses even after use over prolonged periods of time.

Since up to now there is existing no pharmaceutical preparation for the local treatment of herpes viruses which is in the form of an aqueous gel and aqueous gels are advantageous over pasty products such as creams, in the treatment of the external skin because of the cooling effect produced by aqueous gels and because the treated area of the infected skin within short gets dry, it is a further object of the present invention to provide such a pharmaceutical preparation for the local herpes therapy active over such prolonged periods of time which pharmaceutical prepartion is in the form of an aqueous gel.

The pharmaceutical preparation according to the present invention comprises as active principle silver sulfadiazine in combination with one or several non-ionic surfactants in a suitable readily spreadable base material compatible to the human skin, in particular such a base material which represents a non-ionic, hydrophilic aqueous gel.

In particular, the pharmaceutical prepartion according to the present invention consists of 0.05 to 10 % by weight of silver sulfadiazine, 0.3 to 23 % by weight of the non-ionic surfactant or a mixture of several such surfactants and 99.65 to 67.00 % by weight of the readily spreadable base material compatible to the human skin, such that the sum of the above components with 100 % by weight represents the pharmaceutical preparation according to the present invention.

The pharmaceutical preparation according to the present invention preferably comprises two of the non-ionic surfactants. The one of these two non-ionic surfactants is selected from the group of such surfactants having in its molecule only ether groupments between hydrocarbon radicals, one or several thereof possibly are substituted by one or several hydroxy groups and preferably are substituted by one single hydroxy group. Most preferably only one of said hydrocarbon radicals is substituted by one hydroxyl group. The other such non-ionic surfactant is selected from the group of such products having in its molecule ether and ester groupments between the hydrocarbon radicals, one or several of such hydrocarbon radicals being substituted with one or several hydroxy groups. The content by weight of these two non-ionic surfactants in the pharmaceutical preparation according to the present invention preferably relates 1:10 to 1:1, preferably 1:5 to 1:3. In this mixture of the particular two non-ionic surfactants, the non-ionic surfactant selected from the group having only ether groupments between the hydrocarbon radicals represents the larger amount.

The total amount of this mixture of non-ionic surfactants in the pharmaceutical preparation according to the present invention corresponds to the above given content.

The best and longest enduring antiherpes activity accompanied with the best compatibility of the pharmaceutical preparation according to the present invention is obtained and is therefore particularly preferred if the pharmaceutical preparation comprises as third active principle zinc sulphate $ZnSO_4$ in the combination of active agents, in particular in the presence of the above mixture of the two particular non-ionic surfactants. If so, the pharmaceutical preparation of the present invention consists of 0.05 to 10 % by weight of silver sulfadiazine, 0.6 to 13 % by weight of the non-ionic surfactant or mixture of several such non-ionic surfactants, 0.5 to 10 % by weight of zinc sulphate and 98.85 to 67 % by weight of the readily spreadable base material compatible to the human skin. In particular and preferably, the pharmaceutical preparation according to the present invention consists of 0.05 to 10 % by weight of silver sulfadiazine, 0.5 to 10 % by weight of the non-ionic surfactant selected from the group of such surfactants having only ether groupments between hydrocarbon radicals, 0.1 to 3 % by weight of the non-ionic surfactant selected from the group consisting of such surfactants with ether and ester groupments between hydrocarbon radicals, 0.5 to 10 % by weight of zinc sulphate and 98.85 to 67 % by weight of the readily spreadable base material compatible to the human skin. Particularly preferred pharmaceutical preparations according to the invention consist of 2 to 5 % by weight of silver sulfadiazine, 2 to 5 % by weight of the non-ionic surfactant selected from the group of such surfactants having in its molecule solely ether groupments, 0.5 to 2 % by weight of the non-ionic surfactants selected from the group consisting of such surfactants having in its molecule both ether and ester groupments, 0.5 to 2 % by weight of zinc sulphate and 95 to 86 % by weight of the readily spreadable base material compatible to the human skin.

In a most prefer embodiment of the present invention where the pharmaceutical preparation according to the present invention is in the form of an aqueous gel, the readily spreadable base material compatible to the human skin represents a non-ionic hydrophilic aqueous gel.

Suitable non-ionic surfactants for instance are nonylphenoxypolyethoxyethanol (Nonoxynol-9), p-diisobutylphenoxypolyethoxyethanol (Triton X-100), Polyoxyethylen-(10)-oleic ether (Brij-97); sorbitanmono- and -triesters of saturated and unsaturated fatty acids such as lauric acid, palmitic acid, stearic acid or oleic acid (tradename SPAN[R] of Atlas Chemie GmbH); polyoxyethyl derivatives of sorbitan esters, so-called polysorbates such as sorbimacrogollaurate, -palmitate, -stearate, -tristearate, -oleate, -trioleate and the like (known under the tradename TWEEN[R] of ICI America Inc.). Other suitable surfactants are different ethanolamine and isopropanolamine fatty acid condensates (known under the tradename Onyxol[R] of Messrs. Onyx).

Suitable non-ionic hydrophilic aqueous gels are such gels comprising small amounts of a hydroxyalkyl or alkyl cellulose derivative having 1 to 4 preferably 1 or 2 carbon atoms in the hydroxyalkyl and alkyl groups. Such non-ionic hydrophilic aqueous gels for instance consist of 2.5 parts by weight of hydroxyethylcellulose, 10.0 parts by weight of glycerol and 87.5 parts by weight of water or 4.0 parts by weight of methylcellulose 300, 10.0 parts by weight of glycerol and 86.0 parts by weight of water. Methylcellulose 300 represents a methylcellulose derivative having about 300 glucose units in the methylcellulose molecule. These particular compositions of non-ionic hydrophilic aqueous gels conform to DAB (Deutsches Arzneimittelbuch).

Useful as non-ionic hydrophilic aqueous gels are also the so-called liposome products as they are known for instance from W. Raab, Ärztliche Kosmetologie 18, 213 (1988).

The pharmaceutical preparations according to the present invention are characterized by the fact that they retain their high effectivity even after prolonged application. Up to now no undesired side effects or allergic reactions have been observed. By the quick drying of the attacked area of the human skin, the pharamceutical preparation according to the present invention in the form of an aqueous gel produces already a first phase in a curative process and a highly desirable result. The effectivity is great already after a very short period of time. The significant antibacterial and antiviral activity of the component silver sulfadiazine to herpes viruses in this combination of active components and in particular when combined with the preferred non-ionic hydrophilic aqueous gel base material, fully develops and hereby avoids secondary bacterial infections and fully attacks the viral infection. The combination of active agents according to the present invention, in particular in further combination with the particular non-ionic hydrophilic aqueous gel as readily spreadable base material compatible with the skin, allows to avoid any a bacterial superinfection and allows the treatment of such an already existing superinfection.

The following examples further illustrate the present invention.

| Example 1 |  |
| --- | --- |
| The following components are mixed in usual manner: |  |
| Silver sufadiazine | 0.25 g |
| Zinc sulphate | 0.05 g |
| Nonoxynol 9 | 0.50 g |
| Tween-20 | 0.10 g |
| Gel base material adding up to of the complete preapration. | 10.00 g |

The gel base material is a mixture consisting of 2.5 parts by weight of hydroxyethyl cellulose, 10.0 parts by weight of glycerol and 87.5 parts by weight of water.

| Example 2 |  |
| --- | --- |
| The following components are mixed in usual manner. |  |
| Silver sufadiazine | 0.50 g |
| Zinc sulphate | 0.05 g |
| Nonoxynol 9 | 0.50 g |
| Tween-20 | 0.10 g |
| Gel base material adding up to of the complete preparation. | 10.00 g |

The gel base material is the same mixture comprising hydroxyethyl cellulose as described in Example 1.

| Example 3 |  |
| --- | --- |
| The following components are mixed in usual manner. |  |
| Silver sufadiazine | 0.25 g |
| Zinc sulphate | 0.05 g |
| Nonoxynol 9 | 0.50 g |
| Tween-20 | 0.10 g |
| Gel base material adding up to of the complete preparation. | 10.00 g |

The gel base material is a mixture consisting of 4.0 parts by weight of methylcellulose 300, 10.0 parts by weight of glycerol and 86.0 parts by weight of water.

The pharmaceutical products according to the above examples should not be stored at temperatures above 25°C.

## Claims

1. Pharmaceutical preparation for the local herpes therapy comprising a readily spreadable base material compatible to the skin containing silver sulfadiazine, characterized in that the pharmaceutical preparation in combination with silver sulfadiazine comprises one or several non-ionic surfactants.

2. Pharmaceutical preparation according to claim 1, characterized in that it comprises 0.05 to 10 % by weight of silver sulfadiazine, 0.3 to 23 % by weight of the non-ionic surfactant or the mixture of several such non-ionic surfactants and 99.65 to 67.00 % by weight of the readily spreadable base material compatible to the human skin, 100 % by weight of the above components making the total of the above pharmaceutical preparation.

3. Pharmaceutical preparation according to claim 1 or 2, characterized in that it comprises two non-ionic surfactants, the one non-ionic surfactant being selected from the group consisting of the non-ionic surfactants having in its molecule solely ether groupments between hydrocarbon radicals, one or several of said hydrocarbon radicals possibly are substituted by one or several, preferably one hydroxy group, while the other non-ionic surfactant is selected from the group consisting of the non-ionic surfactants having in its molecule ether and ester components between the hydrocarbon radicals, one or several of said hydrocarbon radicals being substituted by one or several hydroxy groups.

4. Pharmaceutical preparations according to claim 3, characterized in that the share in the total weight of the two non-ionic surfactants present is from 1:10 to 1:1, the non-ionic surfactant having in its molecule solely ether components between hydrocarbon radicals is present in the larger amount.

5. Pharmaceutical preparations according to claim 3, characterized in that the share in the total weight of the mixture of the two non-ionic surfactants is from 1:5 to 1:3, the share of the non-ionic surfactant having solely ether components between hydrocarbon radicals is the larger one.

6. Pharmaceutical preparation according to any of the previous claims, in particular according to claim 3, 4 and 5, charaterized in that the pharmaceutical preparation further comprises zinc sulphate $ZnSO_4$ as third active agent in combination with silver sulfadiazine and the non-ionic surfactant or mixture of several such non-ionic surfactants.

7. Pharmaceutical preparation according to any of the previous claims, characterized in that it consists of 2 to 5 % by weight of silver sulfadiazine, 2 to 5 % by weight of the non-ionic surfactant selected from the group of non-ionic surfactants having in its molecule solely ether components between the hydrocarbon radicals, one or several of said hydrocarbon radicals possibly being substituted by one or several, preferably by one hydroxy group, 0.5 to 2 % by weight of the non-ionic surfactant selected from the group of the non-ionic surfactants having ether and ester components between hydrocarbon radicals, one or several of said hydrocarbon radicals being substituted by one or several hydroxy groups, 0.5 to 2 % by weight of zinc sulphate and 95 to 86 % by weight of the readily spreadable base material compatible the human skin.

8. Pharmaceutical preparation according to any of the previous claims, characterized in that the readily spreadable base material compatible with the human skin is a non-ionic hydrophilic aqueous gel.

9. Pharmaceutical preparation according to claim 8, charaterized in that the non-ionic hydrophilic aqueous gel is a liposome gel.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 376 764  (I.R. SCHMOLKA)<br>* Column 3, lines 13-24,55-66; claims 1,5 *<br>--- | 1-5,7,8 | A 61 K   31/635<br>A 61 K   47/50<br>A 61 K    9/127 |
| X | BE-A- 892 421  (LEK, TOVARNA FARMACEVTSKIH IN KEMICNIH IZDELKOV)<br>* Claims 1,7 *<br>--- | 1-5,7,8 | |
| X | US-A-4 659 700  (D.S. JACKSON)<br>* Column 1, lines 37-44; column 2, lines 3,4; claims *<br>--- | 1-5,7,8 | |
| X | FR-A-2 285 896  (NIPPON K.K.K.)<br>* Page 4, lines 10-26; page 5, lines 4-17; page 6, lines 11-22; claims *<br>--- | 1-8 | |
| X,Y | DE-A-2 804 931  (DR. E. FRESENIUS)<br>* Claims 1-4,6; page 4, paragraphs 3-5; page 5, paragraph 3; page 6, paragraph 3 *<br>--- | 1-8 | |
| X | US-A-3 761 590  (C.L. FOX)<br>* Table 1; column 4, lines 14-62; claims 1-6 *<br>--- | 1-5,7,8 | TECHNICAL FIELDS SEARCHED (Int. Cl.5)<br><br>A 61 K |
| D,Y | CUTIS, vol. 38, December 1986, pages 363-365; L.F. MONTES et al.: "Response of varicella zoster virus and herpes zoster to silver sulfadiazine"<br>* Page 1 *<br>--- | 1-8 | |
| P,X | EP-A-0 326 145  (K. MINNINGER)<br>* Whole document *<br>----- | 1-9 | |

The present search report has been drawn up for all claims

| Place of search | Date of completion of the search | Examiner |
|---|---|---|
| THE HAGUE | 09-11-1989 | BERTE M.J. |

EPO FORM 1503 03.82 (P0401)